(19) Europäisches Patentamt
European Patent Office
Office européen des brevets

(11) Veröffentlichungsnummer: 0 146 862
A2

(12) # EUROPÄISCHE PATENTANMELDUNG

(21) Anmeldenummer: 84115107.9

(22) Anmeldetag: 10.12.84

(51) Int. Cl.[4]: C 07 D 409/04
C 07 D 409/14, C 09 K 19/34

(30) Priorität: 21.12.83 DE 3346175

(43) Veröffentlichungstag der Anmeldung: 03.07.85 Patentblatt 85/27

(84) Benannte Vertragsstaaten: CH DE FR GB LI

(71) Anmelder: Merck Patent Gesellschaft mit beschränkter Haftung
Frankfurter Strasse 250
D-6100 Darmstadt(DE)

(72) Erfinder: Eidenschink, Rudolf, Dr.
Kornblumenstrasse 1
D-6115 MWnster(DE)

(72) Erfinder: Scheuble, Bernhard, Dr.
Am Grenzweg 18
D-6146 Alsbach(DE)

(54) **Pyridylthiophene.**

(57) Flüssig-kristalline Verbindungen, enthaltend den strukturellen Bestandteil -Py-Th-,
worin
Py einen Pyridin-2,5-diyl-ring und
Th einen Thiophen-2,4- oder -2,5-diyl-ring
bedeutet, besonders jedoch Pyridylthiophene der Formel I

$$R^1-(A^1-Z^1)_m-(A^2-Z^2)_n-Py-Th-(Z^3-A^3)_p-R^2 \qquad I$$

worin
Py und Th die angegebenen Bedeutungen haben,
$R^1$ und $R^2$ jeweils eine Alkylgruppe mit 1 - 15 C-Atomen, worin auch eine oder zwei $CH_2$-Gruppen durch O-Atome ersetzt sein können; F, C1, Br, CN oder $-O-COR$, einer dieser Reste auch H,
$A^1$, $A^2$ und $A^3$ jeweils unsubstituierte oder durch 1 - 4 F-Atome substituierte 1,4-Phenylen-, 1,4-Cyclohexylen-, 1,3-Dioxan-2,5-diyl, Piperidin-1,4-diyl-, 1,4-Bicyclo-(2,2,2)-octylen-oder Pyrimidin-2,5-diylgruppen, wobei die Cyclohexylengruppe(n) in 1- und oder 4-Stellung durch Alkyl, Alkoxy, fluoriertes Alkyl oder fluoriertes Alkoxy mit jeweils 1 - 5 C-Atomen, F, Cl, Br und oder CN substituiert sein können,
$Z^1$, $Z^2$ und $Z^3$ jeweils -CO-O-, -O-CO-, $-CH_2CH_2-$, $-OCH_2-$, $-CH_2O-$ oder eine Einfachbindung,
(m+n+p) 0,1 oder 2 und
R eine Alkylgruppe mit 1 - 5 C-Atomen
bedeuten,
sowie die Säureadditionssalze der basischen unter diesen Verbindungen eignen sich als Bestandteile flüssigkristalliner Phasen.

EP 0 146 862 A2

Merck Patent Gesellschaft
mit beschränkter Haftung
D a r m s t a d t

Pyridylthiophene

Die Erfindung betrifft flüssig-kristalline Verbindungen, enthaltend den strukturellen Bestandteil -Py-Th-, worin

Py einen Pyridin-2,5-diyl-ring und
Th einen Thiophen-2,4- oder -2,5-diyl-ring

bedeutet, besonders jedoch Pyridylthiophene der Formel I

$$R^1-(A^1-Z^1)_m-(A^2-Z^2)_n-Py-Th-(Z^3-A^3)_p-R^2 \qquad I$$

worin

Py einen Pyridin-2,5-diyl-ring,
Th einen Thiophen-2,4- oder -2,5-diyl-ring,
$R^1$ und $R^2$ jeweils eine Alkylgruppe mit 1 - 15 C-Atomen, worin auch eine oder zwei $CH_2$-Gruppen durch O-Atome ersetzt sein können; F, Cl, Br, CN oder -O-COR, einer dieser Reste auch H,
$A^1$, $A^2$ und $A^3$ jeweils unsubstituierte oder durch 1 - 4 F-Atome substituierte 1,4-Phenylen-, 1,4-Cyclohexylen-, 1,3-Dioxan-2,5-diyl-, Piperidin-1,4-diyl-, 1,4-Bicyclo-(2,2,2)-octylen- oder Pyrimidin-2,5-diyl-gruppen, wobei die Cyclohexylengruppe(n) in 1- und/oder 4-Stellung durch

Alkyl, Alkoxy, fluoriertes Alkyl oder fluoriertes Alkoxy mit jeweils 1 - 5 C-Atomen, F, Cl, Br und/oder CN substituiert sein können,

$Z^1$, $Z^2$ und $Z^3$ jeweils -CO-O-, -O-CO-, $-CH_2CH_2-$, $-OCH_2-$, $-CH_2O-$ oder eine Einfachbindung,

(m+n+p) 0,1 oder 2 und

R eine Alkylgruppe mit 1 - 5 C-Atomen

bedeuten, sowie die Säureadditionssalze der basischen unter diesen Verbindungen.

Der Einfachheit halber bedeuten im folgenden "Phe" eine 1,4-Phenylengruppe, "Cy" eine 1,4-Cyclohexylengruppe, "Dio" eine 1,3-Dioxan-2,5-diylgruppe, "Bic" eine Bicyclo-(2,2,2)-octylengruppe, "Pip" eine Piperidin-1,4-diylgruppe, und "Pyr" eine Pyrimidin-2,5-diylgruppe, wobei diese Gruppen unsubstituiert oder wie oben in der Definition von $A^1$, $A^2$ und $A^3$ angegeben substituiert sein können.

Die Verbindungen der Formel I können wie ähnliche Verbindungen als Komponenten flüssigkristalliner Dielektrika verwendet werden, insbesondere für Displays, die auf dem Prinzip der verdrillten Zelle, dem Guest-Host-Effekt, dem 2-Frequenz-Verfahren, der elektrisch kontrollierten Doppelbrechung oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Dielektrika geeignet sind. Diese Aufgabe wurde durch die Bereitstellung der Verbindungen der Formel I gelöst.

Es wurde gefunden, daß die Verbindungen der Formel I als Komponenten flüssigkristalliner Dielektrika vorzüglich geeignet sind. Insbesondere sind mit ihrer Hilfe stabile flüssigkristalline Phasen mit sehr unterschiedlicher dielektrischer Anisotropie und damit in weiten Grenzen variabler Schwellen- bzw. Steuerspannung elektrooptischer Effekte, variabler optischer Anisotropie und vergleichsweise niedriger Viskosität herstellbar.

Überraschenderweise zeigte sich beim Zusatz von Verbindungen der Formel I zu Mischungen mit positiver dielektrischer Anisotropie, daß selbst größere Zusätze (z.B. von 30 %) von Verbindungen der Formel I mit negativer dielektrischer Anisotropie die Schwellenspannung nur unwesentlich erhöhen. Völlig unerwartet tritt gleichzeitig eine erhebliche Verbesserung der Kennliniensteilheit der Mischung auf, so daß Verbindungen des Typs I als besonders vorteilhaft geeignete Substanzen zur Herstellung von flüssig-kristallinen Mischungen mit steiler Kennlinie anzusehen sind. Sie ermöglichen damit die Entwicklung hoch multiplexbarer Mischungen.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können

aber auch Verbindungen der Formel I flüssigkristallinen Basismaterialien aus anderen Verbindungsklassen zugesetzt werden, um beispielsweise die dielektrische und/ oder optische Anisotropie oder den Mesophasenbereich eines solchen Dielektrikums zu ändern. Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie ausreichend stabil.

Gegenstand der Erfindung sind somit die Verbindungen der Formel I sowie ein Verfahren zu ihrer Herstellung, dadurch gekennzeichnet, daß man eine Verbindung der Formel II mit einer Verbindung der Formel III

$$R^1-(A^1-Z^1)_m-(A^2-Z^2)_n-Py-Q^1 \qquad Q^2-Th-(Z^3-A^3)_p-R^2$$

II　　　　III

worin

entweder　der eine der Reste $Q^1$ und $Q^2$ ein Äquivalent eines Metallatoms oder die Gruppe MgHal, der andere dieser Reste F, Cl, Br oder J und

Hal　Cl, Br oder J

oder der eine der Reste $Q^1$ und $Q^2$ eine Diazoniumsalz- oder eine N-Nitroso-acylaminogruppe und der andere dieser Reste H bedeutet.

und Py, Th, $R^1$, $A^1$, $A^2$, $A^3$, $Z^1$, $Z^2$, $Z^3$, m, n und p die in Anspruch 2 angegebene Bedeutung haben, umsetzt

oder daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt, oder daß man zur Herstellung von Estern der Formel I (worin $R^1$ und/oder $R^2$ -O-COR bedeuten und/oder worin $Z^1$ und/oder $Z^2$ und/oder $Z^3$ -CO-O- oder -O-CO- bedeuten) eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Dioxanderivaten der Formel I (worin $A^1$ und/oder $A^2$ und/oder $A^3$ 1,3-Dioxan-2,5-diyl bedeuten) einen entsprechenden Aldehyd mit einem entsprechenden Diol umsetzt,

oder daß man zur Herstellung von Nitrilen der Formel I (worin $R^1$ und/oder $R^2$ CN bedeuten und/oder worin $A^1$ und/oder $A^2$ und/oder $A^3$ durch mindestens eine CN-Gruppe substituiert ist) ein entsprechendes Carbonsäureamid dehydratisiert oder ein entsprechendes Carbonsäurehalogenid mit Sulfamid umsetzt,

oder daß man zur Herstellung von Ethern der Formel I (worin $R^1$ und/oder $R^2$ eine Alkylgruppe bedeutet, worin eine oder zwei $CH_2$-Gruppen durch O-Atome ersetzt sind und/oder $Z^1$ und/oder $Z^2$ und/oder $Z^3$ eine $-OCH_2-$ oder $-CH_2O-$Gruppe ist) eine entsprechende Hydroxyverbindung verethert,

und/oder daß man zur Herstellung von Chlor- oder Bromverbindungen der Formel I (worin $R^1$ und/oder $R^2$ Cl oder Br bedeuten) eine entsprechende Verbindung der Formel I (worin $R^1$ und/oder $R^2$ H bedeutet) mit einem Chlorierungs- oder Bromierungsmittel behandelt,

und/oder daß man gegebenenfalls eine Chlor- oder Bromverbindung der Formel I (worin $R^1$ und/oder $R^2$ Cl oder Br bedeuten und/oder worin $A^1$ und/oder $A^2$ und/oder $A^3$ durch mindestens ein Chlor- oder Bromatom substituiert ist) mit einem Cyanid umsetzt,

und/oder daß man gegebenenfalls eine Base der Formel I durch Behandeln mit einer Säure in eines ihrer Säureadditionssalze umwandelt,

oder daß man gegebenenfalls eine Verbindung der Formel I aus einem ihrer Säureadditionssalze durch Behandeln mit einer Base freisetzt.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt

an mindestens einer Verbindung der Formel I sowie Flüssigkristall-Anzeigeelemente und elektrooptische Anzeigeelemente, die derartige Phasen enthalten.

Vor- und nachstehend haben Py, Th, $R^1$, $R^2$, $A^1$, $A^2$, $A^3$, $Z^1$, $Z^2$, $Z^3$, m, n, p, R, $Q^1$, $Q^2$ und Hal die angegebene Bedeutung, sofern nicht ausdrücklich etwas anderes vermerkt ist.

Die Verbindungen der Formel I umfassen Verbindungen der Teilformeln Ia (mit zwei Ringen), Ib und Ic (mit jeweils drei Ringen) sowie Id und Ie (mit vier Ringen):

| | |
|---|---|
| $R^1$-Py-Th-$R^2$ | Ia |
| $R^1$-$A^1$-$Z^1$-Py-Th-$R^2$ | Ib |
| $R^1$-Py-Th-$Z^3$-$A^3$-$R^2$ | Ic |
| $R^1$-$A^1$-$Z^1$-Py-Th-$Z^3$-$A^3$-$R^2$ | Id |
| $R^1$-$A^1$-$Z^1$-$A^2$-$Z^2$-Py-Th-$R^2$ | Ie. |

Bevorzugt sind insbesondere Verbindungen der Teilformel Ia. Die weiterhin bevorzugten Teilformeln Ib und Ic umfassen Verbindungen der Teilformeln Iba bis Ibf sowie Ica bis Icf:

| | |
|---|---|
| $R^1$-Phe-$Z^1$-Py-Th-$R^2$ | Iba |
| $R^1$-Cy-$Z^1$-Py-Th-$R^2$ | Ibb |
| $R^1$-Dio-$Z^1$-Py-Th-$R^2$ | Ibc |
| $R^1$-Pip-$Z^1$-Py-Th-$R^2$ | Ibd |
| $R^1$-Bic-$Z^1$-Py-Th-$R^2$ | Ibe |
| $R^1$-Pyr-$Z^1$-Py-Th-$R^2$ | Ibf |
| $R^1$-Py-Th-$Z^3$-Phe-$R^2$ | Ica |
| $R^1$-Py-Th-$Z^3$-Cy-$R^2$ | Icb |
| $R^1$-Py-Th-$Z^3$-Dio-$R^2$ | Icc |
| $R^1$-Py-Th-$Z^3$-Pip-$R^2$ | Icd |
| $R^1$-Py-Th-$Z^3$-Bic-$R^2$ | Ice |
| $R^1$-Py-Th-$Z^3$-Pyr-$R^2$ | Icf |

Darunter sind diejenigen der Formeln Iba, Ibb, Ica und Icb besonders bevorzugt.

In den Verbindungen der vor- und nachstehenden Formeln bedeuten $R^1$ und $R^2$ vorzugsweise Alkyl, ferner Alkoxy (insbesondere, wenn diese Reste an einer Phe-Gruppe stehen) oder eine andere Oxaalkylgruppe.

$A^1$, $A^2$ und $A^3$ sind bevorzugt Cy oder Phe, ferner bevorzugt Dio oder Pip; bevorzugt enthält die Verbindung der Formel I nicht mehr als einen der Reste Dio, Pip, Bic oder Pyr.

Cy ist bevorzugt eine unsubstituierte 1,4-Cyclohexylengruppe, ferner eine 1-X-1,4-cyclohexylengruppe, die keine weiteren Substituenten trägt und worin X Alkyl, Alkoxy, fluoriertes Alkyl oder fluoriertes Alkoxy mit jeweils 1 - 5 C-Atomen, F, Cl, Br oder CN bedeutet. Bevorzugt ist X eine CN-, $CH_3$-, $CH_3O$- oder $CF_3$-Gruppe.

$Z^1$, $Z^2$ und $Z^3$ sind bevorzugt Einfachbindungen, in zweiter Linie bevorzugt -CO-O- oder -O-CO-Gruppen.

Jeder einzelne der Parameter m, n und p kann 0,1 oder 2 bedeuten. Ihre Summe ist auf jeden Fall ebenfalls 0,1 oder 2, vorzugsweise 0.

Falls $R^1$ und/oder $R^2$ Alkylreste bedeuten, in denen auch eine ("Alkoxy" bzw. "Oxaalkyl") oder zwei ("Alkoxyalkoxy" bzw. "Dioxaalkyl") $CH_2$-Gruppen durch O-Atome ersetzt sein können, so können sie geradkettig oder verzweigt sein. Vorzugsweise sind sie geradkettig, haben 2, 3, 4, 5, 6 oder 7 C-Atome und bedeuten demnach bevorzugt

Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy, Tetradecoxy, Pentadexoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formeln I sowie Ia bis Icf mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

In den Resten R und X sind die Alkylgruppen bzw. Alkoxygruppen ebenfalls vorzugsweise geradkettig und bedeuten insbesondere Methyl oder Ethyl, ferner Propyl, Butyl oder Pentyl, X auch Methoxy oder Ethoxy, ferner Propoxy, Butoxy oder Pentoxy.

Unter den Verbindungen der Formeln I sowie Ia bis Icf sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln If bis Izm:

| | |
|---|---|
| $R^1$-Phe-Py-Th-$R^2$ | If |
| $R^1$-Phe-CO-O-Py-Th-$R^2$ | Ig |
| $R^1$-Phe-O-CO-Py-Th-$R^2$ | Ih |
| $R^1$-Phe-$CH_2CH_2$-Py-Th-$R^2$ | Ii |
| $R^1$-Phe-O-$CH_2$-Py-Th-$R^2$ | Ij |
| $R^1$-Phe-$CH_2$-O-Py-Th-$R^2$ | Ik |
| $R^1$-Cy-Py-Th-$R^2$ | Il |
| $R^1$-Cy-CO-O-Py-Th-$R^2$ | Im |
| $R^1$-Cy-O-CO-Py-Th-$R^2$ | In |
| $R^1$-Cy-$CH_2CH_2$-Py-Th-$R^2$ | Io |
| $R^1$-Cy-O-$CH_2$-Py-Th-$R^2$ | Ip |
| $R^1$-Cy-$CH_2$-O-Py-Th-$R^2$ | Iq |
| $R^1$-Dio-Py-Th-$R^2$ | Ir |
| $R^1$-Pip-Py-Th-$R^2$ | Is |
| $R^1$-Bic-Py-Th-$R^2$ | It |
| $R^1$-Pyr-Py-Th-$R^2$ | Iu |
| $R^1$-Phe-Phe-Py-Th-$R^2$ | Iv |
| $R^1$-Py-Th-Phe-$R^2$ | Iw |

| | |
|---|---|
| $R^1$-Py-Th-CO-O-Phe-$R^2$ | Ix |
| $R^1$-Py-Th-O-CO-Phe-$R^2$ | Iy |
| $R^1$-Py-Th-$CH_2CH_2$-Phe-$R^2$ | Iz |
| $R^1$-Py-Th-O-$CH_2$-Phe-$R^2$ | Iza |
| $R^1$-Py-Th-$CH_2$-O-Phe-$R^2$ | Izb |
| $R^1$-Py-Th-Cy-$R^2$ | Izc |
| $R^1$-Py-Th-CO-O-Cy-$R^2$ | Izd |
| $R^1$-Py-Th-O-CO-Cy-$R^2$ | Ize |
| $R^1$-Py-Th-$CH_2CH_2$-Cy-$R^2$ | Izf |
| $R^1$-Py-Th-O-$CH_2$-Cy-$R^2$ | Izg |
| $R^1$-Py-Th-$CH_2$-O-Cy-$R^2$ | Izh |
| $R^1$-Py-Th-Dio-$R^2$ | Izi |
| $R^1$-Py-Th-Pip-$R^2$ | Izj |
| $R^1$-Py-Th-Bic-$R^2$ | Izk |
| $R^1$-Py-Th-Pyr-$R^2$ | Izl |
| $R^1$-Py-Th-Phe-Phe-$R^2$ | Izm |

In den Verbindungen der vorstehend genannten Formeln kann die Gruppe Cy einen Substituenten X enthalten, der in 1- oder 4-Stellung stehen kann. So umschließen z.B. die Verbindungen der Formel Il solche der nachstehenden Teilformeln Il' und Il":

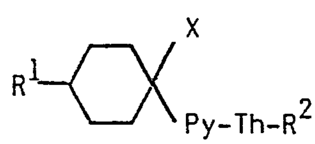

Il'

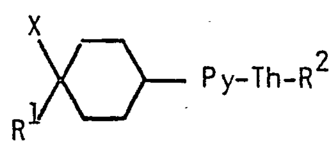

Il"

(wobei der Cyclohexanring zusätzlich einen weiteren Substituenten X in der gegenüberliegenden Stellung (4- bzw. 1-Stellung) des Cyclohexanrings sowie 1 bis 4 weitere F-Atome tragen kann).

In diesem Sinne sind weiterhin Verbindungen der angegebenen Formeln I sowie Ib bis Ie, Ibb, Icb, Il bis Iq und Izc bis Izh besonders bevorzugt, in denen der Rest Cy jeweils

(1) 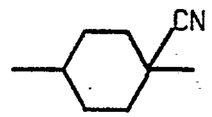 oder

(2) 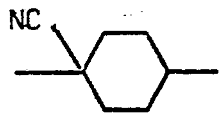 bedeutet.

Dabei sind diejenigen Stereoisomeren bevorzugt, in denen die die Reste $R^1$ bzw. $R^2$ tragenden Gruppen in trans-Stellung zueinander stehen, während der Substituent X in cis-Stellung zu der gegenüberstehenden Gruppe steht. So sind z.B. die nachstehenden Stereoisomeren der Verbindungen der Formel Il' bevorzugt:

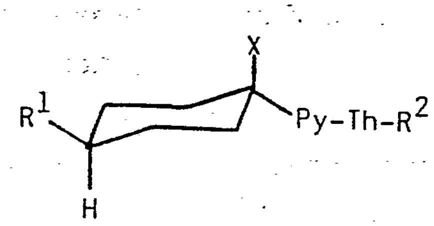

Die vorstehend genannten Verbindungen der Formeln I sowie Ia bis Izm umschließen jeweils die beiden möglichen stellungsisomeren Pyridinderivate, diejenigen der Formel I z.B. die 2-[$R^1$-($A^1$-$Z^1$)$_m$-($A^2$-$Z^2$)$_n$]-5-[Th-($Z^3$-$A^3$)$_p$-$R^3$]-pyridine und die 5-[$R^1$-($A^1$-$Z^1$)$_m$-($A^2$-$Z^2$)$_n$]-2-[Th-($Z^3$-$A^3$)$_p$-$R^3$]-pyridine, diejenigen der Formel Ia dementsprechend die 2-$R^1$-5-(Th-$R^2$)-pyridine und die 5-$R^1$-2-(Th-$R^2$)-pyridine.

Diejenigen der vorstehend genannten Formeln, die eine oder mehrere der Gruppen Dio, Pip und/oder Pyr enthalten, umschließen jeweils die beiden möglichen 2,5- bzw. 1,4-Stellungsisomeren. So umschließt beispielsweise die Teilformel Ibc die 2-$R^1$-5-($Z^1$-Py-Th-$R^2$)-1,3-dioxane und die 2-($Z^1$-Py-Th-$R^2$)-5-$R^1$-1,3-dioxane, die Teilformel Ibd die 1-$R^1$-4-($Z^1$-Py-Th-$R^2$)-piperidine und die 1-($Z^1$-Py-Th-$R^2$)-4-$R^1$-piperidine.

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Organometallverbindung der Formel II ($Q^1$ = M, worin M ein Äquivalent eines Metallatoms, vorzugsweise Li, oder die Gruppe MgHal bedeutet) mit einer Halogenverbindung der Formel III ($Q^2$ = F, Cl, Br oder J) oder indem man eine Halogenverbindung der Formel II ($Q^1$ = F, Cl, Br oder J) mit einer Organometallverbindung der Formel III ($Q^2$ = M) umsetzt, zweckmäßig in Gegenwart eines inerten Lösungsmittels, z.B.

eines Ethers wie Diethyl- oder Diisopropylether, Tetrahydrofuran (THF) oder Dioxan und/oder eines Kohlewasserstoffes wie Hexan oder Cyclohexan bei Temperaturen zwischen etwa 0 und 100 °. Die Ausgangsstoffe sind entweder bekannt, oder sie können in Analogie zu bekannten Stoffen hergestellt werden, z.B. durch Metallierung bzw. Halogenierung von Verbindungen der Formel II ($Q^1$ = H) bzw. III ($Q^2$ = H), die Halogenverbindungen auch aus entsprechenden Aminen über die Diazoniumsalze nach den Methoden von Sandmeyer bzw. Schiemann.

Die Verbindungen der Formel I sind weiterhin erhältlich, indem man ein Diazoniumsalz der Formel II ($Q^1$ = Diazoniumsalzgruppe) oder ein N-Nitroso-acylamin der Formel II ($Q^1$ = N-Nitroso-acylaminogruppe) mit einer Verbindung der Formel III ($Q^2$ = H) oder indem man ein Diazoniumsalz der Formel III ($Q^2$ = Diazoniumsalzgruppe) oder ein N-Nitroso-acylamin der Formel III ($Q^2$ = N-Nitroso-acylaminogruppe) mit einer Verbindung der Formel II ($Q^1$ = H) umsetzt, zweckmäßig unter Verwendung eines Überschußes der Verbindung II ($Q^1$ = H), z.B. Pyridin, oder der Verbindung III ($Q^2$ = H), z.B. Thiophen, als Lösungsmittel bei Temperaturen zwischen etwa -10 und 80 °. Als N-Nitrosoacylamine eignen sich z.B. N-Nitroso-alkanoylamine, worin die Alkanoylgruppe 2 -6 C-Atome hat, z.B. N-Nitrosoacetamide oder N-Nitroso-isobutyramide. Die Bedingungen dieser Umsetzungen können in verschiedener Weise variiert werden, wie es z.B. in Organic Reactions, l.c., Band II, Seiten 224 - 261 (1944) beschrieben ist. Die Ausgangsstoffe sind entweder bekannt oder sie können in Analogie zu bekannten Stoffen nach an sich bekannten Methoden hergestellt werden, die N-Nitrosoacylamine z.B. durch Acylierung entsprechender Amine und anschließende Nitrosierung.

Die Verbindungen der Formel I können auch hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanrings einen Cyclohexanonring und/oder an Stelle einer $-CH_2$-Gruppe eine -CO-Gruppe und/oder an Stelle eines H-Atoms eine freie oder eine funktionell (z.B. in Form ihres p-Toluolsulfonats) abgewandelte OH-Gruppe enthalten.

Ketone können z. B. nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120 $^{o}$) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200 $^{o}$) zu den entsprechenden Verbindungen der Formel I, die Alkylgruppen und/oder $-CH_2CH_2$-Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100 °. Doppelbindungen können (auch in Gegenwart von CN-Gruppen!) mit $NaBH_4$ oder Tributylzinnhydrid in Methanol hydriert werden; so entstehen z.B. aus 1-Cyancyclohexenderivaten die entsprechenden Cyclohexanderivate.

Ester der Formel I ($R^1$ und/oder $R^2$ = -O-COR oder $Z^1$ und/oder $Z^2$ = -CO-O- oder -O-CO-) können auch durch Veresterung entsprechender Carbonsäuren der Formeln R-COOH, $R^1-(A^1-Z^1)_m-A^2-COOH$, $R^1-(A^1-Z^1)_m-Py-Th-COOH$, $HOOC-(A^2-Z^2)_n-Py-Th-(Z^3-A^3)_p-R^2$ oder $HOOC-A^3-R^2$ (oder ihrer reaktionsfähigen Derivate) mit Alkoholen bzw. Phenolen der Formeln $HO-(A^1-Z^1)_m-(A^2-Z^2)_n-Py-Th-(Z^3-A^3)_p-R^2$, $R^1-(A^1-Z^1)_m-(A^2-Z^2)_n-Py-Th-(Z^3-A^3)_p-OH$, $HO-A^3-R^2$, $R^1-A^1-OH$ oder $R^1-(A^1-Z^1)_m-A^2-OH$ (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride der Formeln $R-CO-O-COCH_3$, $R^1-A^1-CO-O-COCH_3$ usw., Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. -phenolate in Betracht, z.B. die entsprechenden Natrium- oder Kaliumalkoholate bzw. -phenolate.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50 $^{o}$ und +250 $^{o}$, vorzugsweise zwischen -20 $^{o}$ und +80 $^{o}$. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem

basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25 ° und +20 °.

Dioxanderivate der Formel I (worin mindestens eine der Gruppen $A^1$, $A^2$ und/oder $A^3$ eine 1,3-Dioxan-2,5-diyl-Gruppe bedeutet) werden zweckmäßig durch Reaktion eines entsprechenden Aldehyds, z.B. der Formeln $R^1-(A^1-Z^1)_m-CHO$, $R^1-(A^1-Z^1)_m-(A^2-Z^2)_n-Py-Th-Z^3-CHO$, $O=CH-Z^1-(A^2-Z^2)_m-Py-Th-(Z^3-A^3)_p-R^2$ oder $O=CH-(Z^3-A^3)_p-R^2$ (oder eines seiner reaktionsfähigen Derivate) mit einem entsprechenden 1,3-Diol z.B. der Formeln $(HOCH_2)_2CH-(A^2-Z^2)-Py-Th-(Z^3-A^3)_p-R^2$, $(HOCH_2)_2-R^2$, $R^1-CH(CH_2OH)_2$ oder $R^1-(A^1-Z^1)_m-(A^2-Z^2)_n-Py-Th-Z^3-CH(CH_2OH)_2$ (oder einem seiner reaktionsfähigen Derivate) hergestellt, vorzugsweise in Gegenwart eines inerten Lösungsmittels wie Benzol oder Toluol

und/oder eines Katalysators, z.B. einer starken Säure wie Schwefelsäure, Benzol- oder p-Toluolsulfonsäure, bei Temperaturen zwischen etwa 20 ° und etwa 150 °, vorzugsweise zwischen 80 ° und 120 °. Als reaktionsfähige Derivate der Ausgangsstoffe eignen sich in erster Linie Acetale z.B. der Formeln $R^1-(A^1-Z^1)_m-CH(OR^3)_2$, $R^1-(A^1-Z^1)_m-(A^2-Z^2)_n-Py-Th-Z^3-CH(OR^3)_2$, $(R^3O)_2CH-Z^1-(A^2-Z^2)_n-Py-Th-(Z^3-A^3)-R^2$, $(R^3O)_2CH-(Z^3-A^3)_p-R^2$, $R^1-(A^1-Z^1)_m-CH(CH_2O)_2CHR^4$, $R^1-(A^1-Z^2)_n-Py-Th-Z^3-CH(CH_2O_2)_2CHR^4$, $R^4CH(OCH_2)_2CH-Z^1-(A^2-Z^2)_n-Py-Th-(Z^3-A^3)-R^2$ oder $R^4CH(OCH_2)_2CH-(Z^3-A^3)_p-R^2$, worin $R^3$ Alkyl mit 1 - 4 C-Atomen, zwei Reste $R^3$ zusammen auch Alkylen mit 2 oder 3 C-Atomen und $R^4$ H, Alkyl mit 1 - 4 C-Atomen oder Phenyl bedeuten.

Die genannten Aldehyde und 1,3-Diole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester erhältlich.

Zur Herstellung von Nitrilen der Formel I (worin $R^1$ und/oder $R^2$ CN bedeuten und/oder worin $A^1$, $A^2$ und/oder $A^3$ durch mindestens eine CN-Gruppe substituiert ist) können entsprechende Säureamide, z.B. solche, in denen an Stelle eines der Reste $R^1$ oder $R^2$ eine $CONH_2$-Gruppe steht, dehydratisiert werden. Die Amide sind z.B. aus entsprechenden Estern oder Säurehalogeniden durch Umsetzung mit Ammoniak erhältlich.

Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0 ° und 150 ° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80 ° und 150 °, vorzugsweise bei 120 °. Nach üblicher Aufarbeitung kann man direkt die Nitrile isolieren. Ether der Formel I (worin $R^1$ und/oder $R^2$ eine Alkylgruppe bedeutet, worin eine oder zwei $CH_2$Gruppen durch O-Atome ersetzt sind, und/oder worin $Z^1$ und/oder $Z^2$ und/oder $Z^3$ eine $-OCH_2-$ oder eine $-CH_2O$-Gruppe ist) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrigalkoholischer NaOH oder KOH bei Temperaturen zwischen etwa 20 ° und 100 °.

Zur Herstellung von Chlor- oder Bromverbindungen der Formel I (worin $R^1$ und/oder $R^2$ Cl oder Br bedeuten) kann eine entsprechende Verbindung der Formel I (worin $R^1$ und/oder $R^2$ H bedeutet) mit einem Chlorierungs- oder Bromierungsmittel behandelt werden, zweckmäßig mit elementarem Chlor oder Brom in einem inerten Lösungsmittel wie $CCl_4$ bei Temperaturen zwischen 0 und 70 °.

Zur Herstellung von Nitrilen der Formel I (worin $R^1$ und/oder $R^2$ CN bedeuten und/oder worin $A^1$, $A^2$ und/oder $A^3$ durch mindestens eine CN-Gruppe substituiert ist) können auch entsprechende Chlor- oder Bromverbindungen der Formel I (worin $R^1$ und/oder $R^2$ Cl oder Br bedeuten und/oder worin $A^1$, $A^2$ und/oder $A^3$ durch mindestens ein Cl- oder Br-Atom substituiert ist) mit einem Cyanid umgesetzt werden, zweckmäßig mit einem Metallcyanid wie NaCN, KCN oder $Cu_2(CN)_2$, z.B. in Gegenwart von Pyridin in einem inerten Lösungsmittel wie DMF oder N-Methylpyrrolidon bei Temperaturen zwischen 20 ° und 200 °.

Eine Base der Formel I kann mit einer Säure in das zugehörige Säureadditionssalz übergeführt werden. Für diese Umsetzung können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure,

Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und-disulfonsäuren, Laurylschwefelsäure.

Umgekehrt ist es möglich, aus einem Säureadditionssalz einer Verbindung der Formel I die Base der Formel I durch Behandeln mit einer Base freizusetzen, z.B. mit einer starken anorganischen Base wie KOH oder NaOH.

Die erfindungsgemäßen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Dielektrika in Frage kommenden Verbindungen lassen sich durch die Formel IV charakterisieren,

R'-L-G-E-R"　　　　IV

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

G　-CH=CH-　　-N(O)=N-
　-CH=CY-　　-CH=N(O)-
　$-C\equiv C-$　　$-CH_2-CH_2-$
　-CO-O-　　$-CH_2-O-$
　-CO-S-　　$-CH_2-S-$
　-CH=N-　　-COO-Phe-COO-

oder eine C-C-Einfachbindung, Y Halogen, vorzugsweise Chlor, oder -CN, und R' und R'' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemäßen Dielektrika enthalten etwa 0,1 bis 100, vorzugsweise 10 bis 100 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.
Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol.Cryst.Liq.Cryst. Band 24, Seiten 249 - 258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben

Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Beispiel 1

Man löst 8,4 g Thiophen in 300 ml Diethylether und versetzt bei 0 ° unter $N_2$ mit 68 ml einer 1,6 M $C_4H_9Li$-Lösung in Hexan. Nach 2 Std. Rühren gibt man eine Lösung von 16,8 g 2-Fluor-5-pentylpyridin (erhältlich aus 3-Pentylpyridin durch Aminierung zu 2-Amino-5-pentylpyridin und anschließende Schiemann-Reaktion) in 100 ml Diethylether hinzu, erwärmt 2 Std. auf 35 °, gießt auf Eis und erhält nach üblicher Aufarbeitung 2-(5-Pentyl-2-pyridyl)-thiophen.

Analog erhält man

2-(5-Ethyl-2-pyridyl)-thiophen
2-(5-Propyl-2-pyridyl)-thiophen
2-(5-Butyl-2-pyridyl)-thiophen
2-(5-Hexyl-2-pyridyl)-thiophen
2-(5-Heptyl-2-pyridyl)-thiophen
2-(5-Octyl-2-pyridyl)-thiophen
2-(5-Pentadecyl-2-pyridyl)-thiophen.

Bespiel 2

Man löst 24,8 g N-(2-Pentyl-5-pyridyl)-isobutyramid (Kp. 167-171 °/0,27 bar; erhältlich durch Reaktion von 2-Chlor-5-nitropyridin mit Na-2-Butylmalonsäurediethylester zu 2-(5-Nitro-2-pyridyl)-2-butylmalonsäurediethylester, Verseifung und Decarboxylierung zu 2-Pentyl-5-nitropyridin, Hydrierung an 10 %ig. Pd-C in Methanol bei 40-50 ° und 1 bar zu 2-Pentyl-5-aminopyridin und

Acylierung mit Isobuttersäureanhydrid) in einem Gemisch aus 110 ml Essigsäure und 50 ml Acetanhydrid, versetzt mit 49 g wasserfreiem Kaliumacetat und anschließend bei 0 ° mit einer Lösung von 6,9 g Nitrosylchlorid in 27 ml Acetanhydrid. Als Zwischenprodukt entsteht N-Nitroso-N-(2-pentyl-5-pyridyl)-isobutyramid, das nicht isoliert wird. Nach 15. Min. Rühren bei 0 ° wird auf Eis gegossen und fünfmal mit je 140 ml Thiophen extrahiert. Man trennt ab, hält die organische Phase bei 0 °, schlämmt sie mit 22 g $K_2CO_3$ und 35 g $Na_2SO_4$ auf und dekantiert. Es wird auf 50 ° erwärmt, nach dem Ende der Gasentwicklung 2 Std. gekocht, eingedampft und an Kieselgel mit Toluol getrennt. Man erhält 2-(2-Pentyl-5-pyridyl)-thiophen, Kp. 180-185 °/ 0,013 bar, K. -45 °; daneben entsteht wenig 3-(2-Pentyl-5-pyridyl)-thiophen, Kp. 170-180 °/0,015 bar, K. -80 °.

Analog erhält man

2-(2-Propyl-5-pyridyl)-thiophen
3-(2-Propyl-5-pyridyl)-thiophen
2-(2-Butyl-5-pyridyl)-thiophen
3-(2-Butyl-5-pyridyl)-thiophen
2-(2-Hexyl-5-pyridyl)-thiophen
3-(2-Hexyl-5-pyridyl)-thiophen
2-(2-Heptyl-5-pyridyl)-thiophen
3-(2-Heptyl-5-pyridyl)-thiophen
2-(2-Octyl-5-pyridyl)-thiophen
3-(2-Octyl-5-pyridyl)-thiophen
2-(2-Pentyl-5-pyridyl)-5-methyl-thiophen
2-[2-(trans-4-Propylcyclohexyl)-5-pyridyl]-thiophen
2-[2-(trans-4-Pentylcyclohexyl)-5-pyridyl]-thiophen
2-[2-(2-(trans-4-Propylcyclohexyl)-ethyl)-5-pyridyl]-thiophen
2-[2-(2-(trans-4-Methoxymethylcyclohexyl)-ethyl)-5-pyridyl]-thiophen.

Beispiel 3

Man kocht ein Gemisch von 2,73 g 2-Acetyl-4-(2-pentyl-5-pyridyl)-thiophen [F. 72 °; erhältlich durch Reaktion von 2-(2-Pentyl-5-pyridyl)-thiophen mit Acetylchlorid/ $SnCl_4$ in Benzol], 1,6 g 85 %iger Hydrazinhydratlösung, 1,6 g KOH und 8 ml Triethylenglykol 2 Std., erhitzt unter Abdestillieren von Wasser und Hydrazinhydrat auf 195 °, kühlt ab und erhält nach üblicher Aufarbeitung 2-Ethyl-5-(2-pentyl-5-pyridyl)-thiophen, Kp. 180 °/0,07 mbar.

Analog sind durch Reduktion entsprechender Ketone erhältlich:

2-Ethyl-5-(2-propyl-5-pyridyl)-thiophen
2-Ethyl-5-(2-butyl-5-pyridyl)-thiophen
2-Ethyl-5-(2-hexyl-5-pyridyl)-thiophen
2-Ethyl-5-(2-heptyl-5-pyridyl)-thiophen
2-Propyl-5-(2-propyl-5-pyridyl)-thiophen
2-Propyl-5-(2-butyl-5-pyridyl)-thiophen
2-Propyl-5-(2-pentyl-5-pyridyl)-thiophen
2-Propyl-5-(2-hexyl-5-pyridyl)-thiophen
2-Propyl-5-(2-heptyl-5-pyridyl)-thiophen
2-Butyl-5-(2-propyl-5-pyridyl)-thiophen
2-Butyl-5-(2-butyl-5-pyridyl)-thiophen
2-Butyl-5-(2-pentyl-5-pyridyl)-thiophen
2-Butyl-5-(2-hexyl-5-pyridyl)-thiophen
2-Butyl-5-(2-heptyl-5-pyridyl)-thiophen
2-Pentyl-5-(2-propyl-5-pyridyl)-thiophen
2-Pentyl-5-(2-butyl-5-pyridyl)-thiophen
2-Pentyl-5-(2-pentyl-5-pyridyl)-thiophen
2-Pentyl-5-(2-hexyl-5-pyridyl)-thiophen
2-Pentyl-5-(2-heptyl-5-pyridyl)-thiophen

2-Hexyl-5-(2-propyl-5-pyridyl)-thiophen
2-Hexyl-5-(2-butyl-5-pyridyl)-thiophen
2-Hexyl-5-(2-pentyl-5-pyridyl)-thiophen
2-Hexyl-5-(2-hexyl-5-pyridyl)-thiophen
2-Hexyl-5-(2-heptyl-5-pyridyl)-thiophen
2-Heptyl-5-(2-propyl-5-pyridyl)-thiophen
2-Heptyl-5-(2-butyl-5-pyridyl)-thiophen
2-Heptyl-5-(2-pentyl-5-pyridyl)-thiophen
2-Heptyl-5-(2-hexyl-5-pyridyl)-thiophen
2-Heptyl-5-(2-heptyl-5-pyridyl)-thiophen.

Beispiel 4

Man kocht 20,5 g 5-(2-Thienyl)-picolinsäure 1 Std. mit 24 g $SOCl_2$, dampft ein, löst das erhaltene rohe Säurechlorid in 150 ml Toluol, versetzt mit 8 ml Pyridin und 16,7 g cis-4-Cyan-4-propylcyclohexanol (erhältlich durch Alkylierung von 4-Cyancyclohexanol) und kocht 2 Stunden. Nach Abkühlen und üblicher Aufarbeitung erhält man 5-(2-Thienyl)-picolinsäure-(cis-4-cyan-4-propylcyclohexylester).

Analog erhält man mit den entsprechenen Cyclohexanolen:

5-(2-Thienyl)-picolinsäure-(cis-4-methyl-4-propylcyclohexylester)
5-(2-Thienyl)-picolinsäure-(cis-4-methoxy-4-propylcyclohexylester)
5-(2-Thienyl)-picolinsäure-(cis-4-trifluormethyl-4-propylcyclohexylester).

Beispiel 5

Ein Gemisch von 1,2 g 2-Propylpropan-1,3-diol, 2,59 g 5-(2-Pentyl-5-pyridyl)-2-formyl-thiophen (erhältlich durch Formylierung von 5-(2-Pentyl-5-pyridyl)-thiophen nach Vilsmeier), 0,01 g p-Toluolsulfonsäure und 15 ml Toluol wird am Wasserabscheider 3 Std.gekocht, abgkühlt, mit Wasser gewaschen und eingedampft. Man erhält 5-(2-Pentyl-5-pyridyl)-2-(trans-5-propyl-1,3-dioxan-2-yl)-thiophen.

Analog erhält man durch Umsetzung der entsprechenden Aldehyde mit den entsprechenden Diolen:

5-(2-Propyl-5-pyridyl)-2-(trans-5-propyl-1,3-dioxan-2-yl)-thiophen
5-(2-Propyl-5-pyridyl)-2-(trans-5-butyl-1,3-dioxan-2-yl)-thiophen
5-(2-Propyl-5-pyridyl)-2-(trans-5-pentyl-1,3-dioxan-2-yl)-thiophen
5-(2-Propyl-5-pyridyl)-2-(trans-5-hexyl-1,3-dioxan-2-yl)-thiophen
5-(2-Propyl-5-pyridyl)-2-(trans-5-heptyl-1,3-dioxan-2-yl)-thiophen
5-(2-Butyl-5-pyridyl)-2-(trans-5-propyl-1,3-dioxan-2-yl)-thiophen
5-(2-Butyl-5-pyridyl)-2-(trans-5-butyl-1,3-dioxan-2-yl)-thiophen
5-(2-Butyl-5-pyridyl)-2-(trans-5-pentyl-1,3-dioxan-2-yl)-thiophen
5-(2-Butyl-5-pyridyl)-2-(trans-5-hexyl-1,3-dioxan-2-yl)-thiophen
5-(2-Butyl-5-pyridyl)-2-(trans-5-heptyl-1,3-dioxan-2-yl)-thiophen

5-(2-Pentyl-5-pyridyl)-2-(trans-5-butyl-1,3-dioxan-2-yl)-thiophen

5-(2-Pentyl-5-pyridyl)-2-(trans-5-pentyl-1,3-dioxan-2-yl)-thiophen

5-(2-Pentyl-5-pyridyl)-2-(trans-5-hexyl-1,3-dioxan-2-yl)-thiophen

5-(2-Pentyl-5-pyridyl)-2-(trans-5-heptyl-1,3-dioxan-2-yl)-thiophen

5-(2-Hexyl-5-pyridyl)-2-(trans-5-propyl-1,3-dioxan-2-yl)-thiophen

5-(2-Hexyl-5-pyridyl)-2-(trans-5-butyl-1,3-dioxan-2-yl)-thiophen

5-(2-Hexyl-5-pyridyl)-2-(trans-5-pentyl-1,3-dioxan-2-yl)-thiophen

5-(2-Hexyl-5-pyridyl)-2-(trans-5-hexyl-1,3-dioxan-2-yl)-thiophen

5-(2-Hexyl-5-pyridyl)-2-(trans-5-heptyl-1,3-dioxan-2-yl)-thiophen

5-(2-Heptyl-5-pyridyl)-2-(trans-5-propyl-1,3-dioxan-2-yl)-thiophen

5-(2-Heptyl-5-pyridyl)-2-(trans-5-butyl-1,3-dioxan-2-yl)-thiophen

5-(2-Heptyl-5-pyridyl)-2-(trans-5-pentyl-1,3-dioxan-2-yl)-thiophen

5-(2-Heptyl-5-pyridyl)-2-(trans-5-hexyl-1,3-dioxan-2-yl)-thiophen

5-(2-Heptyl-5-pyridyl)-2-(trans-5-heptyl-1,3-dioxan-2-yl)-thiophen.

Beispiel 6

Eine Lösung von 31,6 g 2-(trans-4-Propylcyclohexyl)-5-(5-carbamoyl-2-thienyl)-pyridin (erhältlich aus dem Säurechlorid mit $NH_3$) in 500 ml DMF wird bei 50 ° unter Rühren tropfenweise mit 65 g $POCl_3$ versetzt. Nach weiterem einstündigem Rühren gießt man auf Eis, arbeitet wie üblich auf und erhält 2-(trans-4-Propylcyclohexyl)-5-(5-cyan-2-thienyl)-pyridin.

Analog erhält man durch Wasserabspaltung aus den entsprechenden Amiden:
2-p-Cyanphenyl-5-(2-thienyl)-pyridin
2-(4'-Cyan-4-biphenylyl)-5-(2-thienyl)-pyridin
2-(5-Cyan-2-pyrimidyl)-5-(2-thienyl)-pyridin.

Beispiel 7

Eine Lösung von 37,6 g 1-Pentyl-cis-4-[5-(2-thienyl)-2-pyridyl]-cyclohexan-r-1-carbonylchlorid [erhältlich durch Reaktion von 4-[5-(2-Thienyl)-2-pyridyl)]-cyclohexanon mit Pentyl-Li und nachfolgende Hydrolyse zu 1-Pentyl-4-[5-(2-thienyl)-2-pyridyl]-cyclohexanol, Umsetzung mit K, dann mit $CO_2$ zu 1-Pentyl-cis-4-[5-(2-thienyl)-2-pyridyl]-cyclohexan-r-1-carbonsäure und Reaktion mit $SOCl_2$] und 8 g Sulfamid in 500 ml Tetramethylensulfon wird 4 Std. auf 120 ° erhitzt, eingedampft und wie üblich aufgearbetet. Man erhält r-1-Cyan-1-Pentyl-cis-4-[5-(2-thienyl)-2-pyridyl]-cyclohexan.

Beispiel 8

Ein Gemisch von 15,3 g 2-(2-p-Hydroxyphenyl-5-pyridyl)-thiophen, 6,9 g $K_2CO_3$, 25 g Hexyljodid und 250 ml DMF wird unter Rühren 16 Std. auf 80 ° erhitzt, dann abgekühlt und wie üblich aufgearbeitet. Man erhält 2-(2-p-Hexoxyphenyl-5-pyridyl)-thiophen.

Beispiel 9

Eine Lösung von 32,9 g r-1-Hydroxy-1-pentyl-cis-4-[5-(2-thienyl)-2-pyridyl]-cyclohexan [erhältlich durch Oxydation von 4-[5-(2-Thienyl)-2-pyridyl]-cyclohexanol mit $CrO_3$ zum Keton, Reaktion mit Pentyl-MgBr und Hydrolyse] in 280 ml 1,2-Dimethoxyethan wird mit 4,8 g NaH und 27,8 g $CH_3J$ versetzt. Nach 5 Std. Erhitzen auf 70 ° kühlt man ab, zersetzt mit Wasser, arbeitet wie üblich auf und erhält r-1-Methoxy-1-pentyl-cis-4-[5-(2-thienyl)-2-pyridyl]-cyclohexan.

Beispiel 10

In eine Lösung von 23,1 g 2-(2-Pentyl-5-pyridyl)-thiophen in 40 ml $CCl_4$ werden bei 20 ° 7,3 g Chlor eingeleitet bzw. unter Rühren 17 g Brom zugetropft. Nach 1 Std. Stehen erwärmt man 2 Std. auf 50 °, wäscht mit verdünnter Natronlauge, arbeitet wie üblich auf und erhält 5-Chlor-2-(2-pentyl-5-pyridyl)-thiophen, F. 32 °, K. 18 ° (bzw. 5-Brom-2-(2-pentyl-5-pyridyl)-thiophen).

Analog erhält man durch Halogenierung:

5-Chlor-2-(2-propyl-5-pyridyl)-thiophen
5-Chlor-2-(2-butyl-5-pyridyl)-thiophen
5-Chlor-2-(2-hexyl-5-pyridyl)-thiophen
5-Chlor-2-(2-heptyl-5-pyridyl)-thiophen
5-Brom-2-(2-propyl-5-pyridyl)-thiophen
5-Brom-2-(2-butyl-5-pyridyl)-thiophen
5-Brom-2-(2-hexyl-5-pyridyl)-thiophen
5-Brom-2-(2-heptyl-5-pyridyl)-thiophen.

Beispiel 11

Ein Gemisch aus 26,6 g 5-Chlor-2-(2-pentyl-5-pyridyl)-thiophen, 10 g $Cu_2(CN)_2$, 120 ml Pyridin und 60 ml N-Methylpyrrolidon wird 2 Std. auf 150 ° erhitzt. Man kühlt ab, gibt eine Lösung von 120 g $FeCl_3$ . 6 $H_2O$ in 600 ml 20 %iger Salzsäure hinzu, erwärmt 1,5 Std. unter Rühren auf 70 °, arbeitet wie üblich auf und erhält 2-(2-Pentyl-5-pyridyl)-thiophen-5-carbonitril.

Analog sind aus den entsprechenden Chlor- oder Bromverbindungen erhältlich:

2-(2-Propyl-5-pyridyl)-thiophen-5-carbonitril
2-(2-Butyl-5-pyridyl)-thiophen-5-carbonitril
2-(2-Hexyl-5-pyridyl)-thiophen-5-carbonitril
2-(2-Heptyl-5-pyridyl)-thiophen-5-carbonitril.

Es folgen Beispiele für erfindungsgemäße Dielektrika mit einem Gehalt an mindestens einer Verbindung der Formel I:

Beispiel A

Ein Gemisch aus

21 % 2-(2-Pentyl-5-pyridyl)-thiophen
22 % p-trans-4-Butylcyclohexyl-benzonitril
14 % 4-Ethyl-4'-cyanbiphenyl
18 % 4-Butyl-4'-cyanbiphenyl
16 % 4-Cyan-4'-(trans-4-pentylcyclohexyl)-biphenyl
9 % 4-(trans-4-Pentylcyclohexyl)-4'-(trans-4-propylcyclohexyl)-biphenyl

zeigt K. 62 °.

Beipiel B

Ein Gemisch aus

15 % 2-(2-Pentyl-5-pyridyl)-thiophen-5-carbonitril
15 % p-trans-4-Propylcyclohexyl-benzonitril
11 % p-trans-4-Butylcyclohexyl-benzonitril
21 % p-trans-4-Pentylcyclohexyl-benzonitril
21 % 4-Ethyl-4'-(trans-4-pentylcyclohexyl)-biphenyl
12 % 4-(trans-4-Pentylcyclohexyl)-4'-(trans-4-propylcyclohexyl)-biphenyl
5 % 4-Cyan-4'-(trans-4-pentylcyclohexyl)-biphenyl

zeigt K. 90 °.

Merck Patent Gesellschaft
mit beschränkter Haftung
D a r m s t a d t

## Patentansprüche:

1. Flüssig-kristalline Verbindungen, enthaltend den strukturellen Bestandteil -Py-Th-,
worin
Py einen Pyridin-2,5-diyl-ring und
Th einen Thiophen-2,4- oder -2,5-diyl-ring
bedeutet.

2. Pyridylthiophene der Formel I

$$R^1-(A^1-Z^1)_m-(A^2-Z^2)_n-Py-Th-(Z^3-A^3)_p-R^2 \qquad I$$

worin
Py einen Pyridin-2,5-diyl-ring,
Th einen Thiophen-2,4- oder -2,5-diyl-ring,
$R^1$ und $R^2$ jeweils eine Alkylgruppe mit 1 - 15 C-Atomen, worin auch eine oder zwei $CH_2$-Gruppen durch O-Atome ersetzt sein können; F, Cl, Br, CN oder -O-COR, einer dieser Reste auch H,
$A^1$, $A^2$ und $A^3$ jeweils unsubstituierte oder durch 1 - 4 F-Atome substituierte 1,4-Phenylen-, 1,4-Cyclohexylen-, 1,3-Dioxan-2,5-diyl-, Piperidin-1,4-diyl-, 1,4-Bicyclo-(2,2,2)-octylen- oder Pyrimidin-2,5-diylgruppen, wobei die Cyclohexylengruppe(n) in 1- und/oder 4-Stellung

| | |
|---|---|
| | durch Alkyl, Alkoxy, fluoriertes Alkyl oder fluoriertes Alkoxy mit jeweils 1 - 5 C-Atomen, F, Cl, Br und/oder CN substituiert sein können, |
| $Z^1$, $Z^2$ und $Z^3$ | jeweils -CO-O-, -O-CO-, $-CH_2CH_2-$, $-OCH_2-$, $-CH_2O-$ oder eine Einfachbindung, |
| (m+n+p) | 0,1 oder 2 und |
| R | eine Alkylgruppe mit 1 - 5 C-Atomen |

bedeuten, sowie die Säureadditionssalze der basischen unter diesen Verbindungen.

3. Verfahren zur Herstellung von Pyridylthiophenen der Formel I nach Anspruch 2, dadurch gekennzeichnet, daß man eine Verbindung der Formel II mit einer Verbindung der Formel III

$$R^1-(A^1-Z^1)_m-(A^2-Z^2)_n-Py-Q^1 \qquad Q^2-Th-(Z^3-A^3)_p-R^2$$

II III

worin

| | |
|---|---|
| entweder | der eine der Reste $Q^1$ und $Q^2$ ein Äquivalent eines Metallatoms oder die Gruppe MgHal, |
| | der andere dieser Reste F, Cl, Br oder J und |
| | Hal Cl, Br oder J |
| oder | der eine der Reste $Q^1$ und $Q^2$ eine Diazoniumsalz- oder eine N-Nitroso-acylaminogruppe und |
| | der andere dieser Reste H bedeutet |

und Py, Th, $R^1$, $A^1$, $A^2$, $A^3$, $Z^1$, $Z^2$, $Z^3$, m, n und p die in Anspruch 2 angegebene Bedeutung haben, umsetzt

oder daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,

oder daß man zur Herstellung von Estern der Formel I (worin $R^1$ und/oder $R^2$ -O-COR bedeuten und/oder worin $Z^1$ und/oder $Z^2$ und/oder $Z^3$ -CO-O- oder -O-CO- bedeuten) eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Dioxanderivaten der Formel I (worin $A^1$ und/oder $A^2$ und/oder $A^3$ 1,3-Dioxan-2,5-diyl bedeuten) einen entsprechenden Aldehyd mit einem entsprechenden Diol umsetzt,

oder daß man zur Herstellung von Nitrilen der Formel I (worin $R^1$ und/oder $R^2$ CN bedeuten und/oder worin $A^1$ und/oder $A^2$ und/oder $A^3$ durch mindestens eine CN-Gruppe substituiert ist) ein entsprechendes Carbonsäureamid dehydratisiert oder ein entsprechendes Carbonsäurehalogenid mit Sulfamid umsetzt,

oder daß man zur Herstellung von Ethern der Formel I (worin $R^1$ und/oder $R^2$ eine Alkylgruppe bedeutet, worin eine oder zwei $CH_2$-Gruppen durch O-Atome ersetzt sind und/oder $Z^1$ und/oder $Z^2$ und/oder $Z^3$ eine $-OCH_2-$ oder $-CH_2O-$Gruppe ist) eine entsprechende Hydroxyverbindung verethert,

und/oder daß man zur Herstellung von Chlor- oder Bromverbindungen der Formel I (worin $R^1$ und/oder $R^2$ Cl oder Br bedeuten) eine entsprechende Verbindung der Formel I (worin $R^1$ und/oder $R^2$ H bedeutet) mit einem Chlorierungs- oder Bromierungsmittel behandelt,

und/oder daß man gegebenenfalls eine Chlor- oder Bromverbindung der Formel I (worin $R^1$ und/oder $R^2$ Cl oder Br bedeuten und/oder worin $A^1$ und/oder $A^2$ und/oder $A^3$ durch mindestens ein Chlor- oder Bromatom substituiert ist) mit einem Cyanid umsetzt,

und/oder daß man gegebenenfalls eine Base der Formel I durch Behandeln mit einer Säure in eines ihrer Säureadditionssalze umwandelt,

oder daß man gegebenenfalls eine Verbindung der Formel I aus einem ihrer Säureadditionssalze durch Behandeln mit einer Base freisetzt.

4. Verwendung einer Verbindung nach Anspruch 1 als Komponente flüssigkristalliner Phasen.

5. Verwendung einer Verbindung der Formel I nach Anspruch 2 als Komponente flüssigkristalliner Phasen.

6. Flüssigkristalline Phase, dadurch gekennzeichnet, daß sie mindestens eine Verbindung nach Anspruch 1 enthält.

7. Flüssigkristalline Phase, dadurch gekennzeichnet, daß sie mindestens eine Verbindung der Formel I nach Anspruch 2 enthält.

8. Flüssigkristall-Anzeigeelement, dadurch gekennzeichnet, daß es eine Phase nach Anspruch 6 enthält.

9. Flüssigkristall-Anzeigeelement, dadurch gekennzeichnet, daß es eine Phase nach Anspruch 7 enthält.

10. Elektrooptisches Anzeigeelement, dadurch gekennzeichnet, daß es eine Phase nach Anspruch 6 enthält.

11. Elektrooptisches Anzeigeelement, dadurch gekennzeichnet, daß es eine Phase nach Anspruch 7 enthält.